# EUROPEAN PATENT SPECIFICATION

(11) **EP 0 921 832 B1**
(45) Date of publication and mention of the grant of the patent: **14.01.2004**
(21) Application number: 97933387.9
(22) Date of filing: 11.07.1997
(51) Int. Cl.: A61M 25/00

(54) **METHOD FOR FORMING HIGH COMPLIANCE, HIGH STRENGTH CATHETER BALLOONS USEFUL FOR TREATMENT OF GASTROINTESTINAL LESIONS**
VERFAHREN ZUR HERSTELLUNG VON HOCHFESTEN DILATATIONSBALLONS MIT HOHER COMPLIANCE ZUR BEHANDLUNG GASTROINTESTINALER SCHÄDIGUNGEN
METHODE DE FABRICATION DE BALLONNETS DE CATHETER A COEFFICIENTS D'ELASTICITE ET DE RESISTANCE ELEVES UTILISABLES POUR LE TRAITEMENT DE LESIONS GASTRO-INTESTINALES

(30) Priority: 23.07.1996 US 685338
(43) Date of publication of application: 16.06.1999
(73) Proprietor: Scimed Life Systems, Inc., Maple Grove, Minnesota 55311-1566 (US)
(72) Inventor: WANG, Lixiao, Maple Grove, MN 55369 (US); FOURNIER, Donald, J., Bellevue, WA 98004 (US)
(74) Representative: Graalfs, Edo, Dipl.-Ing.
(86) International application number: PCT/US1997/012074
(87) International publication number: WO 1998/003218

(56) References cited:
- EP-A- 0 439 202
- EP-A- 0 485 903
- EP-A- 0 592 885
- WO-A-95/23619
- WO-A-96/12516
- US-A- 5 348 538

## Description

This application refers to catheter balloons useful for treatment of gastrointestinal lesions.

### Background of the Inventions

Balloons mounted on the distal ends of catheters are widely used in medical treatment. The balloon may be used to widen a vessel into which the catheter is inserted or to force open a blocked vessel. The requirements for strength and size of the balloons vary widely depending on the balloon's intended use and the vessel size into which the catheter is inserted.

Perhaps the most demanding applications for such balloons ar in balloon angioplasty in which catheters are inserted for long distances into extremely small vessels and used to open stenoses of blood vessels by balloon inflation. These applications require extremely thin walled, high strength, relatively inelastic balloons of predictable inflation properties. Thin walls are necessary because the balloon's wall and waist thicknesses limit the minimum diameter of the distal end of the catheter and therefore determine the limits on vessel size treatable by the method and the case of passage of the catheter through the vascular system. High strength is necessary because when the balloon is used to push open a stenosis, the thin wall must not burst under the high internal pressures necessary to accomplish this task. The balloon must have some elasticity so that the inflated diameter can be controlled, enabling the surgeon to vary the balloon's diameter as required to treat individual lesions, but that elasticity must be relatively low so that the diameter is easily controllable. Small variations in pressure must not cause wide variations in diameter. Such angioplasty balloons have nominal diameters in the range of from about 1.25-4.5 mm:

Outside the field of angioplasty, however, relatively high compliant, high strength materials are desirable for some balloons used on esophageal, pyloric, colonic and anastomotic catheters and scopes.

Major advances in the ability to access remote areas within the gastrointestinal tract have allowed endoscopists to reach obstructive lesions previously accessible only via open surgical techniques. There are three primary approaches available to the clinician for endoscopic treatment of gastrointestinal strictures: 1) Mercury bougie dilatation; 2) Over-the-wire passage of tapered dilators; and 3) Balloon dilation.

Of the three, balloon dilatation is the most recently developed modality. Clinical research studies have been conducted to compare the efficacy of the technique to earlier approaches. For example, in one study evaluating Savary-Guillard® Dilators versus balloon dilators for dilatation of benign esophageal strictures, both methods achieved effective dilatation. However, during the 24 month follow-up, 88% of patients treated with Savary dilators required redilations vs 54% of patients in the balloon group. As a result, the researchers concluded that, over the long term, the balloon may provide superior efficacy. Additional studies have clearly documented the convenience, effectiveness and safety of balloon dilatation of strictures.

An important advantage of balloon dilatation over the alternative techniques is that it enables the clinician to dilate remote strictures throughout the GI tract.

An example is the treatment of esophagal achalasia. The esophagus, a hollow, muscular organ that originates at the pharynx and terminates at the stomach, functions to transport food and fluids from the pharynx to the stomach via a complex, neuromuscular response to the act of swallowing. Specifically, the passage of food or fluid from the pharynx into the esophagus stimulates the peristaltic contractions designed to propel the contents forward through the esophagus. Concurrently, the lower esophageal sphincter (LES) at the gastroesophagal junction relaxes allowing the passage of esophagal contents into the stomach. Reflux of stomach contents back into the esophagus is prevented by closure of the LES. Achalasia is a disorder of unknown etiology that disrupts the normal esophageal function. In this disorder, two deficits are present. First, the normal esophagal peristaltic wave is absent. Second, the lower esophageal sphincter does not relax. The result is esophageal dilatation and severe, progressive dysphagia. Treatment for achalasia is aimed at reduction of LES pressure. This is accomplished nonsurgically via forceful balloon dilatation of the sphincter.

Biliary dilatation may also be performed by such balloon catheter dilatation. Biliary strictures may result from variety of processes including postoperative scarring, inflammation, or malignancy. Endoscopic balloon dilatation of these lesions has been shown to be an effective treatment approach.

There therefore is a need for effective devices which permit endoscopic dilatation of lesions throughout the alimentary tract. It is important that the catheters offer first-use effectiveness in an advanced design to permit rapid inflation, deflation and easy scope passage. The balloons for such devices desirably would have a long dilation length, high operating pressure, typically grater than 50 psi (3 atm, 344.7 kPa) and desirably up to 146 psi, (10 atm, 1013 kPa), low withdrawal force and high compliance. For instance, a compliance change is desirable which would allow a balloon having a 1.25-3.0 mm nominal diameter at 3 atm to grow in a generally linear manner at least 0.25 mm, preferably about 0.5 mm, or more as pressure is increased from 3 to 12 atm. For balloons about 3.25-6.0 mm nominal diameter, a growth of at least 1.0 mm over the same range would be desirable. For balloons in the range of about 6-12 mm nominal diameter, a growth of at least 2.0 mm over a 3-10 atm pressure range would be desirable. For even larger diameter balloons, for instance balloons having 3 atm diameters of 12-30 mm, a compliance curve which provides growth of about 3 mm or more, preferably about 4.0 mm or more, over the range of 3 to 9 atm is desirable.

In US 5,348,538, there is described a single layer angioplasty balloon made of material such as PET which follows a stepped compliance curve. The stepped compliance curves of these balloons have a low pressure segment following a first generally linear profile, a transition region, typically in the 8-14 atm range, during which the balloon rapidly expands yielding inelastically, and a higher pressure region in which the balloon expands along a generally linear, low compliance curve. The stepped compliance curve allows a physician to dilate different sized lesions without using multiple balloon catheters.

A polyethylene ionomer balloon with a stepped compliance curve is discloses in EP 540 858.

In WO 95/23619, there are described balloons, useful on angioplasty catheters and similar medical devices, which are made from certain block copolymer materials which provide an unusual combination of compliance, softness and strength properties. The balloons are formed by extrusion. The extruded polymer material is cooled in a deionized water bath maintained at about 18°C.

Block copolymer balloons for balloon catheters, prepared using a particular heat set technique to stabilize the balloon dimensions, are also described in US 5,500,180.

### Summary of the Invention

The invention provides balloons having the desired properties just described. In one aspect, the invention is a method for forming a balloon for a medical device in which a tubing of a thermoplastic polymer material is radially expanded under a first elevated pressure at a first elevated temperature to form the balloon at a first diameter, the thermoplastic polymer material being a block copolymer material and the method including the further step of annealing the balloon at a second elevated temperature less than or equal to the first temperature and a second pressure less than the first elevated pressure for a time sufficient to shrink the formed balloon to a second diameter less than the first diameter. Suitably the second temperature is in the range of 70-100°C, the second pressure is no more than about 50 psi (3.4023 atm, 344.738 kPa), and the time of annealing is sufficient to shrink the balloon so that its diameter at 3 atm pressure is about 90% or less, preferably about 85% or less, of the 3 atm diameter of a correspondingly prepared balloon prepared without said shrinking step.

In some embodiments to improve balloon-to-balloon reproducibility of the process, the balloon may be shrunk at a very low inflation pressure (typically 0-10 psi, 68.9476 kPa) to a nominal diameter below that desired in the final balloon, and then pressurized at a pressure between the shrink pressure and 50 psi (3.4023 atm, 344.738 kPa), at a temperature within the same range within a mold or cylinder which is sized to provide the desired nominal diameter, still below the diameter at which the balloon was initially blown, and suitably 90% or less of the initial blow diameter.

The shrinking process used in the invention is quite different from the heat set technique used in US 5,500,180, in that the process of US 5,500,180 after formation of the balloon heats the balloon under pressure of 100-500 psi (6.8046 - 34.023 atm, 989.476 - 3447.38 kPa) to a temperature above the blowing temperature specifically for the purpose of stabilizing the balloon against shrinkage upon cooling. The present invention is directed to exploitation of shrinkage behavior in order to increase the compliance of the resulting balloon

Balloons made of multiple layers of thermoplastic material, such as coextruded balloons of the types described in US 5,195,969, US 5,270,086, and US 5,478,320, or separately blown dual layer balloons of the types described in US 5,512,051, WO 96/04951 and in US 5,805,600, filed 6 March 1996, may also be employed in the present invention by shrinking the so-formed in accordance with the present invention after is has been formed.

There are described herein balloons particularly suited to dilation of GI lesions of various types which are characterized by unique combinations of balloon diameter, high burst strength and high compliance characteristics.

The high strength, high compliance balloons of the invention also provide excellent rewrap characteristics, in comparison of high strength balloons formed by other processes. Consequently, after treatment of a lesion and deflation of the balloon, the force required to withdraw the balloon catheter from the body is low, especially for catheters designed to pass through endoscopes (TTS).

### Brief Description of the Drawings

Fig. 1 is a schematic side view of a through-the scope dilation catheter of the invention.
Fig. 2 is a schematic side view of a dual lumen over-the wire dilation catheter of the invention.
Fig. 3 is a plot showing compliance curves of balloons prepared in Examples 3 and 4.
Fig. 4 is a plot showing compliance curves of balloons prepared in Example 5.
Fig 5. is a plot showing compliance curves of balloons prepared in Example 6.

### Detailed Description of the Invention

Typically, balloon dilatation catheters for gastrointestinal applications are available in two design options: 1) through-the scope (TTS); and 2) over-the-wire (OTW). Each design offers particular advantages in specific clinical situations.

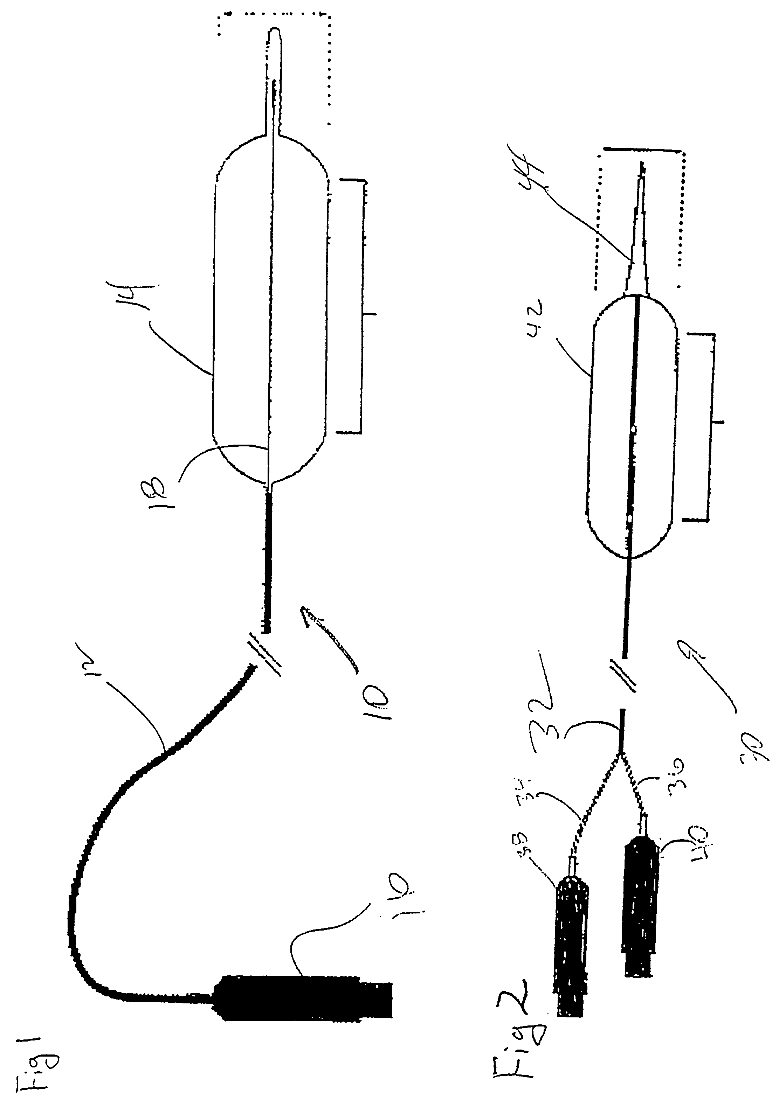

Referring to Fig. 1, a TTS balloon dilatation catheter 10 is shown. Catheter 10 comprises a shaft 12, a balloon 14 near the distal end thereof and an inflation apparatus 16 at the proximal end thereof. Shaft 12 has a strong flexible kink-resistant construction and includes a longitudinal lumen extending therethrough by means of which the balloon 14 may be inflated by the inflation apparatus 16. Catheter 10 is designed for direct passage through the working channel of the endoscope to the site of an obstructive lesion. Because ITS catheters are not passed over a guidewire, the design includes a mechanism for stiffening the shaft enough to allow advancement through the narrow scope channel to the lesion. To achieve this result, TTS catheters suitably incorporate a stiffening stylet 18 within the shaft. This stylet extends from the proximal end of the shaft through the length of the balloon and provides the stiffness required to facilitate passage through an endoscope and enhance appropriate positioning within the lesion.

TTS balloon dilatation catheters are useful when an endoscopist prefers to dilate under direct visualization. With the endoscope placed immediately proximal to the lesion, catheter advancement and balloon inflation can be directly monitored. Scope placement immediately proximal to the balloon also assists in maintaining proper balloon position during inflation. For example, during dilatation of pyloric structures, the balloon has a tendency to slip proximally or distally during inflation. By positioning the scope at the proximal end of the balloon, the endoscopist is able to mechanically block any backward movement during inflation thereby facilitating efficient, effective dilatation.

Fig. 2 illustrates the construction of an OTW balloon dilation catheter 30. Catheter 30 includes a shaft 32 which has a strong flexible kink-resistant construction and incorporates a double-lumen design. This design provides a lumen for guidewire passage and a lumen for balloon inflation. At the proximal end of catheter 30 the lumens of shaft 32 divide into separate portions 34, 36 which communicate respectively to balloon inflation apparatus 38 and to guidewire control apparatus 40. A balloon 42 is mounted on the shaft 32 near the distal end of catheter 30. Catheter 30 may incorporate a soft flexible tip 44 to provide less traumatic advancement through tortuous or narrow strictures. Tip 44 may be radiopaque to assist in fluoroscopic positioning of the catheter at the lesion site.

OTW balloon dilatation catheters are the preferred design option when extremely tortuous of difficult anatomy is encountered. In these cases, the ability to track the catheter over a previously placed guidewire enhances the clinician's ability to precisely position the balloon for effective dilatation. Further, for colonic lesions distal to scope access, a fluoroscopically-guided, OTW placement technique can be used to achieve appropriate positioning.

With OTW balloon dilatation catheters, the endoscopist typically monitors balloon inflation fluoroscopically rather than under direct visualization. Therefore, these dilators incorporate radiopaque markers to aid in accurate positioning. These catheters are designed to be used with guidewires and are available in a variety of shaft and balloon lengths and diameters. This allows the endoscopist to select a catheter appropriate for each lesion.

With regard to balloon size selection, for successful dilatation, the balloon must provide effective radial force against the entire length of the stricture. In Figs. 1 and 2 the balloon diameter is indicated by dimension A and the dilation length indicated by dimension B. The balloon should be long enough to dilate the entire length of the stricture. The inflated outer diameter should affect the desired degree of dilatation.

The length of the catheter shaft required varies with the location of the structure. For example, catheters 180 cm in length allow access to esophageal and pyloric lesions, while a catheter 240 cm in length is required for colonic strictures.

With the exception of Achalasia Balloon Dilators which are inflated with air, balloons are primarily inflated with water, saline or a contract/saline mixture. The latter is used with OTW balloon catheters to enhance visualization. It is important not to exceed the maximum inflation pressure specified for any particular balloon catheter. Surpassing this pressure could lead to balloon rupture.

During a procedure the balloon is fully inflated to the desired dilatation diameter. Dilatation force is applied for as long as necessary to achieve desired results. Using an inflation apparatus equipped with a pressure gauge, the balloons of the present invention provide a substantially wider range of stricture diameters which may be treated with a single catheter. After treatment, the deflated balloons of the invention provide substantially reduced resistance to withdrawal of the catheter from the body compared to prior art high strength balloons made for instance from biaxially oriented PET. In some TTS applications measured withdrawal force through the scope for catheters bearing balloons of the invention has been found to be only about 1/2 of the withdrawal force for corresponding catheters bearing PET balloons of similar burst strength.

For esophageal balloon dilatation TTS catheters, a balloon of approximately 8 cm length (dimension B), having an outer diameter (dimension A) of 6 to 20 mm is suitable. The preferred length of the catheter is 180 cm.

For pyloric balloon dilatation TTS catheters, a balloon of approximately 5.5 cm length balloon, having an outer diameter of 6 to 20 mm is suitable. The preferred length of the catheter is 180 cm.

For colonic balloon dilatation TTS catheters, a balloon of approximately 5.5 cm length balloon, having an outer diameter of 6 to 20 mm is suitable. The preferred length of the catheter is 240 cm.

For anastomotic balloon dilatation TTS catheters, a balloon of approximately 8 cm length balloon, having an outer diameter of 20-30 mm is suitable. The preferred length of the catheter is 240 cm.

Balloon materials are thermoplastic block copolymers, preferably characterized as follows:
the block copolymer is made up of hard segments of a polyester or polyamide and soft segments of polyether;
the polyester hard segments are polyesters of an aromatic dicarboxylic acid and a C₂-C₄ diol,
the polyamide hard segments are polyamides of C₆ or higher, preferably C₁₀-C₁₂, carboxylic acids and C₆ or higher, preferably C₁₀-C₁₂, organic diamines or of C₆ or higher, preferably C₁₀ -C₁₂, aliphatic ω-amino-α-acids, and
the polyether soft segments are polyechers of C₂-C₁₀, preferably C₁-C₆ diols;and
the percentage by weight of the block polymer attributable to the hard segments is between about 50% and about 98%. Suitably the block copolymer has a low flexural modulus, namely less than 150,000 psi (10,206.9 atm, 1.03421e + 006 kPa), preferably less than 120,000 psi (8,165.52 atm, 827371 kPa), and has a hardness, Short: D scale, of greater than 60. From such polymers, balloons having very high compliance profiles can be prepared with high wall strength. The low flexural modulus contributes to a softer feel found with the balloons of the invention, compared to those made of other high strength polymer material.

The preferred balloons made with the method of the invention are made of polyamide/polyether block copolymers. The polyamide/polyether block copolymers are commonly identified by the acronym PEBA (polyether block amide). The polyamide and polyether segments of these block copolymers may be linked through amide linkages, however, most preferred are ester linked segmented polymers, *i.e.* polyamide/polyether polyesters. Such polyamide/polyether/polyester block copolymers are made by a molten state polycondensation reaction of a dicarboxylic pulyamide and a polyether diol. The result is a short chair polyester made up of blooks of polyamide and polyether. The polyamide and polyether blocks are not miscible. Thus the materials are characterized by a two phase structure: one is a thermoplastic region that is primarily polyamide and the other is elastomer region that is rich in polyether. The polyamide segments are semicrystalline at room temperature. The generalized chemical formula for these polyester polymers may be represented by the following formula: 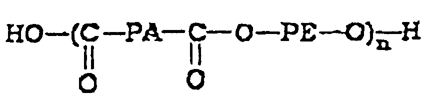 in which PA is a polyamide segment, PE is a polyether segment and the repeating number n is between 5 and 10.

The polyamide segments are suitably aliphatic polyamides, such as nylons 12, 11, 9, 6, 6/12, 6/11,6/9, or 6/6. Most preferably they are nylon 12 segments. The polyamide segments may also be based on aromatic polyamides but in such case significantly lower compliance characteristics are to be expected. The polyamide segments are relatively low molecular weight, generally within the range of 500-8,000, more preferably 2,000-6,000, most preferably about 3,000-5,000.

The polyether segments are aliphatic polyethers having at least 2 and no more than 10 linear saturated aliphatic carbon atoms between ether linkages. More preferably the ether segments have 4-6 carbons between ether linkages, and most preferably they are poly(tetramethylene ether) segments. Examples of other polyethers which may be employed in place of the preferred tetramethylene ether segments include polyethylene glycol, polypropylene glycol, poly(pentamethylene ether) and poly(hexamethylene ether). The hydrocarbon portions of the polyether may be optionally branched. An example is the polyether of 2-ethylhexane diol. Generally such branches will contain no more than two carbon atoms. The molecular weight of the polyether segments is suitably between about 400 and 2,500, preferably between 650 and 1000.

The weight ratio of polyamide to polyether in the polyamide/polyether polyesters used in the invention desirably should be in the range of 50/50 to 98/2, preferably between 60/30 and 92/08, more preferably, between 70/30 and 90/10.

Polyamide/polyether polyesters are sold commercially under the Pebax® trademark by Atochem North America, Inc., Philadelphia PA. Examples of suitable commercially available polymers are the Pebax® 33 series polymers with hardness 60 and above, Shore D scale, especially Pebax® 7233, 7033 and 6333. These polymers are made up of nylon 12 segments and poly(tetramethylene ether) segments in different weight ratios and segment lengths.

It is also possible to use other PEBA polymers with the physical properties specified herein and obtain similar compliance, strength and softness characteristics in the finished balloon.

As an alternative to polyamide elastomers, it is also possible to utilize polyester/polyether segmented block copolymers and obtain similar balloon properties. Such polymers are made up of at least two polyester and at least two polyether segments. The polyether segments are the same as previously described for the polyamide/polyether biock copolymers useful in the invention. The polyester segments are polyesters of an aromatic dicarboxylic acid and a two to four carbon diol.

Suitable dicarboxylic acids used to prepare the polyester segments of the polyester/polyether block copolymers are ortho-, meta- or para- phthalic acid, napthalenedicarboxylic acid or meta-terphenyl-4,4'-dicarboxylic acids.

Preferred polyester/polyether block copolymers are poly(butylene terephthalate)-*block*-poly(tetramethylene oxide) polymers such as Arnitel EM 740, sold by DSM Engineering Plastics. Hytrel polymers: soid by DuPont which meet the physical and chemical specifications set out herein can also be used.

It is preferred that the block copolymers have a hardness, Shore D scale, of at least 60 and a flexural modulus of no more than about 150,000, in order to obtain optimal strength, compliance and softness characteristics. Preferably the Shore D hardness is in the range of 65-75 and the flexural modulus is in the range of 50,000-120,000. The preferred polymers useful in the invention are also characterized by a high ultimate elongation of about 300% or higher and an ultimate tensile strength of at least 6,000 psi (408.276 aim, 41368.5 kPa).

Other thermoplastic polymer materials which may be used to prepare balloons in accordance with the invention include blends of rigid and flexible polymers; polyurethanes which have flexible portions, typically derived from polyester or polyether polyols and rigid portions, typically derived from diisocyanates; random copolymers of rigid and flexible monomers: aliphatic polyketones; polysulfides such as PPS. (polyphenylenesulfide); polyamides, for instance C₆ or higher polyamides which are saturated with water, C₁₁ or higher polyamide homopolymers regardless of water content, and polyamide copolymers of linear and branched monomer units; and other polymers or polymer blends which are known in the art as thermoplastic elastomers. Specific additional thermoplastic polymer products which are considered suitable include polyurethane/polycarbonate blend or block copolymers sold under the trademarks, TEXIN IPU by Bayer Corp and TECOTHANE by Thermedics Inc. and polyurethanes sold under the trademark PELLETHANE by Dow Chemical Co. As previously mentioned, multilayer balloon structures formed by concentric coextrusion of different thennoplastic polymers, or by sequential concentric blowing of separate tubing parisons of different materials, may also be employed.

Manufacture of balloons of the invention is started with an extruded tubing of the thermoplastic polymer material.

The balloon, prior to its being shrunk, may be manufactured in accordance with known techniques such as described in U.S. Patent 5,556,383, entitled Block Copolymer Elastomer Catheter Balloons, incorporated herein by reference, which corresponds to WO 95/23619, and late published WO 97/17089, entitled Method of Balloon Formation by Cold Drawing/Necking. Multi-staged blowing techniques as described in US 5 542 851 filed 17 Feb. 1994 may also be employed.

The balloon shrinking process is similar to that described in US 5,348,538 for balloons of non-compliant material such as PET. However, the balloons of the invention are desirably constructed by blowing the balloon from a block copolymer or other polymer material as described above and then shrinking the balloon to a greater extent than was done in the specific illustrative examples of US 5,348,538. The amount of shrinkage is controlled by the pressure maintained in the balloon during annealing and the temperature and time of the annealing. For a balloon made from Pebax® 7233, the blowing pressure is suitably in the range 200-400 psi (13.6092 - 27.2184 atm, 1378.95 - 2757.9 kPa), and temperature is suitably in the range of 90-100°C, and the annealing pressure is in the range of 0-50, preferably 1-30 psi (6.89476 - 206,343 kPa) at 70-100°C for 3-30 seconds.

By shrinking until the balloon, at 3 atm pressure, provides a diameter of about 90% or less, preferably 85% or less, and more preferably about 65%-75% of the diameter of a correspondingly prepared balloon, at 3 atm pressure, which does not undergo shrinking, a very steep compliance curve is obtained which is more generally linear, the greater the shrinkage. Burst, strength is not substantially affected by the shrinking step. However the shrinking step causes the compliance curve to start from a lower point so that overall the balloon is much more compliant. In this manner the comparatively high strength of the block copolymer material is made accessible to medical device applications where high compliance is also desirable.

To improve balloon-to-balloon reproducibility of the process, the balloon may be shrunk at a very low inflation pressure (typically 0-30 psi, 0 - 2.04138 atm, 0 - 206.843 kPa) to a nominal diameter below that desired in the final balloon, and then pressurized at a pressure between the shrink pressure and 50 psi (3.4023 atm, 344.7 kPa), at a temperature within the same range within a mold or cylinder- which is sized to provide the desired nominal diameter, still below the diameter at which the balloon was initially blown, and suitably 90% or less of the initial blow diameter. Example 10 is illustrative of this technique.

The invention is illustrated by the following non-limiting examples.

### Example 1 (Comparative Example)

Pebax® 7233 tubes with dimensions of 0.105 inch (2.667mm) ID (inner diameter) x 0.140 (3.556 mm) OD (outer diameter) are cold-drawn at a very low temperature, approximately in the range of-100°C to -20° C as follows. A screw driven Stretching machine with a pair of pneumatic grippers is used to stretch the tubing. The center portion of the tube is cooled by directly spraying liquid N₂ on the center portion. Five 30 mm length balloons are blown at 95°C in a 16 mm diameter mold using a blowing pressure of 260 psi (1792.64 kPa) and a tension of 160 grams. The average double wall thickness of the balloons was 0.00282 inch (0.07162,8 mm). The burst pressure was 9.2 atm. The compliance from 3 atm to 5 atm was 42% and from 3 atm to burst pressure was 11.7%.

### Example 2

Five balloons were prepared as in Example 1. The balloons, while inflated at about 5 psi (34.4738 kPa) pressure, were shrunk by dipping in a 80°C water bath for 5 minutes. The average double wall thickness after shrinking was 0.00429 inch (0.108966 mm). The average burst pressure of the balloons was 9.4 atm. The compliance from 3 atm to 5 atm was 15% and from 3 atm to burst pressure was 39%

### Example 3 (Comparative Example)

Pebax® 7233 tubes with dimensions of 0.0264 inch (0.67056 mm) ID x 0.0464 (1.17856 mm) OD (outer diameter) are cold-drawn as in Example 1. A 4 mm balloon is blown at 95°C in a mold using a blowing pressure of 450 psi (3102.64 kPa) and a tension of 300 grams, The burst pressure was 22 atm.

### Example 4

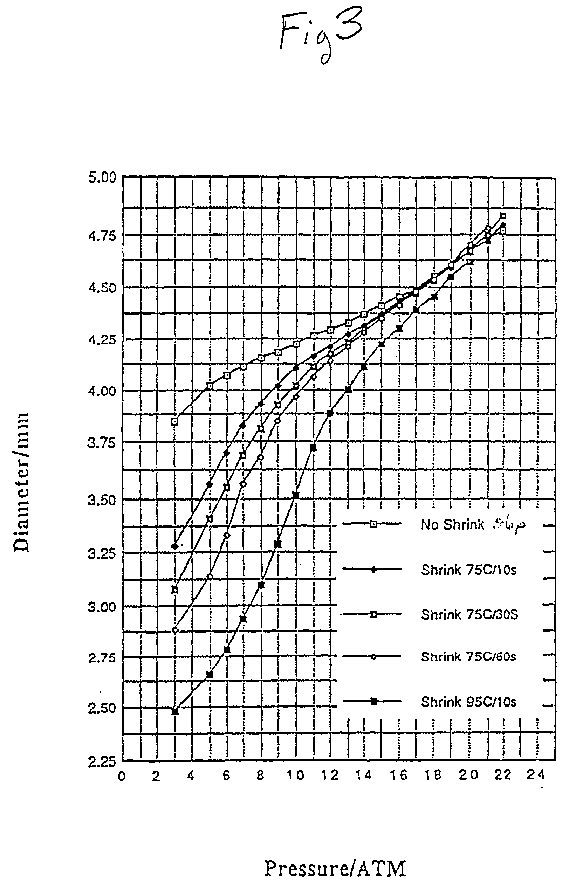

The process of Example 2 is repeated except that the shrinking step is performed by annealing the balloons while inflated to 2 atm pressure in water baths under different conditions, namely: 75 ° C for 10 seconds; 75 °C for 30 seconds; 75 °C for 60 seconds; and 95°C for 10 seconds. Compliance curves for the balloons of Examples 3 and 4 are plotted in Figure 3.

### Example 5

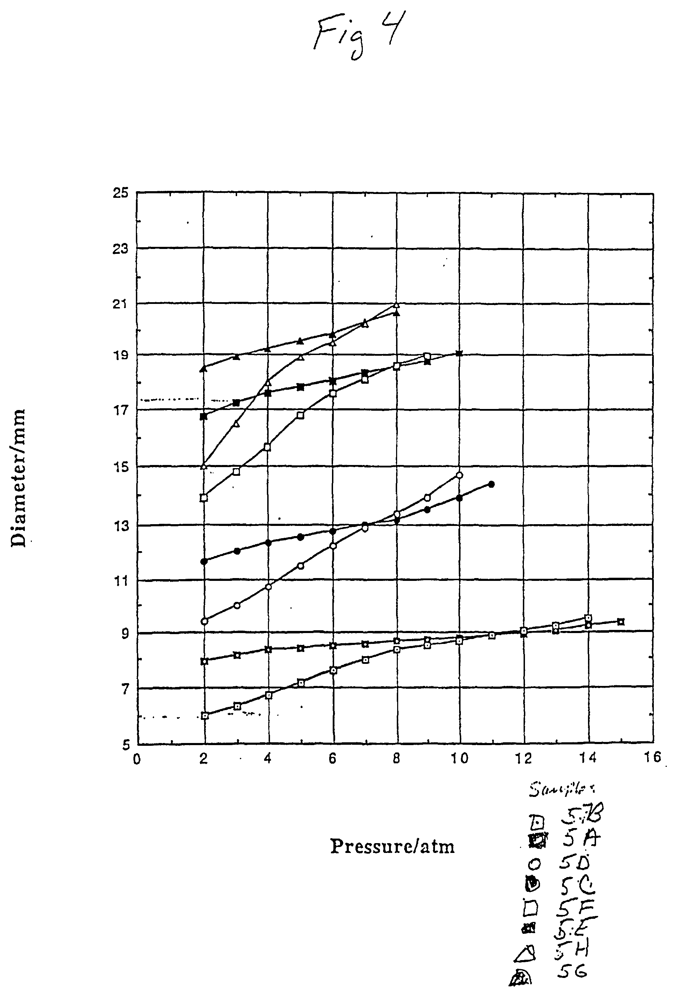

Balloons were prepared as in Examples 1 and 2 using Pebax 7033 polymer and the conditions specified in Table 1. The compliance results are summarized in Table 1 and are plotted in Figure 4.

### Example 6

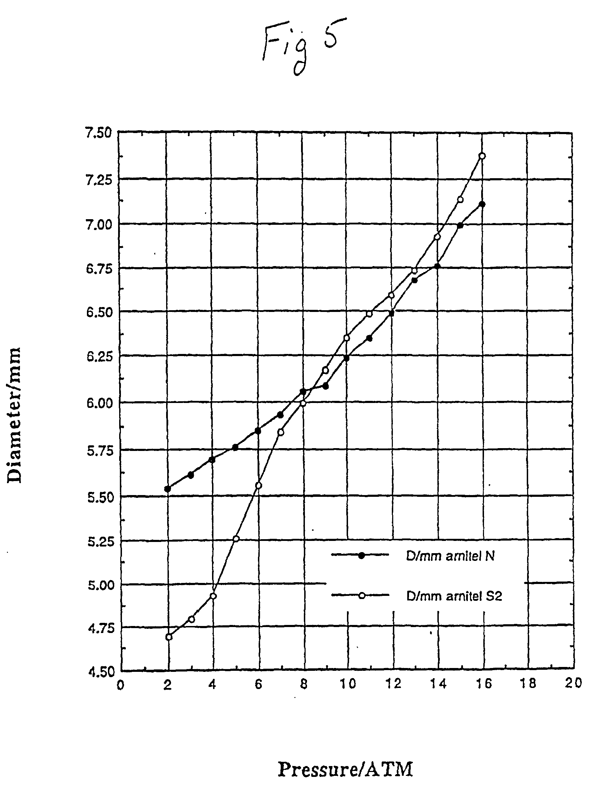

Balloons were made from Arnitel EM 740 polymer cubing by stretching tubing as specified in Table 2 at room temperature at a stretch ratio of 4.2 and then blowing the balloon from the stretched tubing under the conditions specified in Table 2. The compliance results are summarized in Table 2 and are plotted in Figure 5.

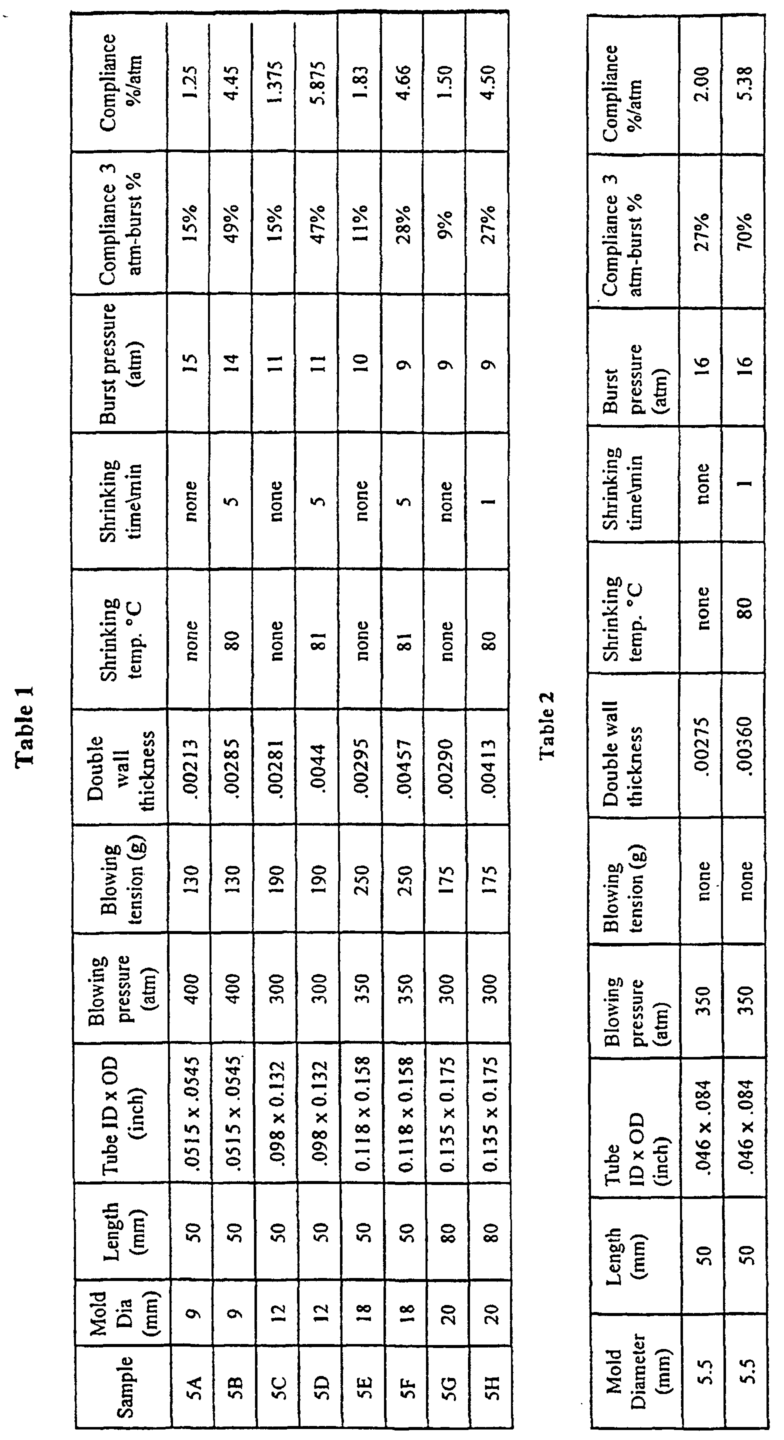

As can be seen from Figs 3-5, the balloons prepared utilizing the shrinking step have very high compliance profiles, in addition to high wall strength. The shrinking step causes, the compliance curve to start from a lower point so that overall the balloon is much more compliant. In this manner the comparatively high strength of the block copolymer material is made accessible to medical device applications where high compliance is also desirable.

### Example 9 (Comparative Example)

Extruded Pebax 7233 tubes with dimensions of 0.0509 inch (1.29286 mm) ID x 0.0729 inch (1.85166 mm) OD were prepared. Five 55 mm length balloons were blown at a temperature of 95°C in an 8 mm diameter mold using a blowing pressure of 430 psi (3102.64 kPa) and a tension of 150 grams. The average double wall thickness of the balloons was 0.00174 inch (0.044196 mm). The average burst pressure was 13.6 atm. The compliance from 3 atm to 10 atm was 9.0 %, and from 3 atm to burst pressure was 15%.

### Example 10

Five balloons were prepared as in Example 9. The balloons while at a tension and inflated at atmospheric pressure were shrunk by dipping in an 85°C water bath for 2 minutes. The balloons were then each inserted into a 5.6 mm ID glass tube, replaced in the 85° bath and pressurized at 30 psi (206.843 kPa) for 2 minutes. The resulting balloons had an average double wall thickness of 0.00233 inch (0.059182 mm). an average burst pressure of 12.6 mm, a compliance from 3 atm to 10 atm of 36% and a compliance from 3 atm to burst of 44%.

The above disclosure is intended to be illustrative and not exhaustive. These examples and description will suggest many variations and alternatives to one of ordinary skill in this art All these alternatives and variations are intended to be included. within the scope of the attached claims. Those familiar with the art may recognize other equivalents to the specific embodiments described herein which equivalent are also intended to be encompassed by the claims attached hereto.

## Claims

1. A method of forming a balloon for a medical device, wherein a tubing of a thermoplastic polymer material is radially expanded under a first pressure at a first temperature to form the balloon at a first diameter, and the method including the further step of annealing the balloon at a second temperature above ambient and less than the first temperature and a second pressure for a time sufficient to shrink the formed balloon to a second diameter less than the first diameter,
**characterized in that**
- the thermoplastic polymer material is a block copoylmer material,
- the second pressure is above ambient and less than the first pressure, and
- the method further comprising the step of pressurizing the balloon in a fixed diameter form, said fixed diameter being greater than said second diameter but no more than 90% of said first diameter, at a pressure above the annealing pressure but no more than 50 psi (206.843 kPa) and a temperature not less than said second temperature and not greater than said first temperature for a time to provide the balloon with a final diameter at 3 atm inflation pressure which is greater than said second diameter but not more than 90% of said first diameter.

2. The method as in claim 1, wherein the second pressure is no more than 20 psi (137.895 kPa).

3. The method as in claim 2, wherein the second pressure is within the range of 1-10 psi (6.89476 - 68.9476 kPa).

4. The method as in one of the claims 1 to 3, wherein the first temperature is within the range of 90-100°C and the second temperature is within the range of 70-100°C.

5. The method as in one of the claims 1 to 4, wherein the block copolymer is made up of soft segments a polyether and hard segments of a polyester or a polyamide.

6. The method as in claim 5, wherein the polyether soft segments are polyethers of C₂-C₁₀ diols.

7. The method as in claim 5 or 6, wherein the hard segments are polyesters of an aromatic dicarboxylic acid and a C₂-C₄ diols.

8. The method as in claim 5 or 6, wherein the hard segments are polyamides chosen from the group consisting of a combination of C₆ or higher carboxylic acids and C₆ or higher organic diamines and C₆ or higher, aliphatic ω-amino-α-acids.

9. The method as in one of the preceding claims, wherein:
the first blowing pressure is above ambient and greater than 50 psi (206.843 kPa) at the first temperature to form the balloon to have the first diameter at 3 atm inflation pressure,
the second temperature is above ambient and less than or equal to the first temperature, and at a second pressure which in the range of 0-20 psi (0 - 137895 kPa), for a time sufficient to shrink the formed balloon to have the second diameter at 3 atm inflation pressure which is less than 90% of the first diameter.

10. A method as in claim 9 wherein the balloon is pressurized in the fixed diameter form for a time such that said final diameter is 85% or less of said first diameter.

11. A method as in claim 9 wherein the balloon is pressurized in the fixed diameter form for a time such that said final diameter is 65-75% of said first diameter.

## Patentansprüche

1. Verfahren zur Ausbildung eines Ballons für eine medizinische Vorrichtung, wobei ein Schlauch aus einem thermoplastischen Polymermaterial unter einem ersten Druck bei einer ersten Temperatur radial ausgedehnt wird, um den Ballon mit einem ersten Durchmesser auszubilden, und das Verfahren den weiteren Schritt des Temperns des Ballons bei einer zweiten Temperatur, die über der Umgebungstemperatur und unter der ersten Temperatur liegt, und unter einem zweiten Druck über einen Zeitraum umfaßt, der zum Schrumpfen des ausgebildeten Ballons auf einen zweiten Durchmesser ausreicht, der kleiner als der erste Durchmesser ist,
**dadurch gekennzeichnet, daß**
- das thermoplastische Polymermaterial ein Blockcopolymer-Material ist,
- der zweite Druck über dem Umgebungsdruck und unter dem ersten Druck liegt, und
- das Verfahren weiter den Schritt des Unterdrucksetzens des Ballons in der Form eines festen Durchmessers, wobei der feste Durchmesser größer als der zweite Durchmesser ist, jedoch nicht mehr als 90 % des ersten Durchmessers beträgt, bei einem Druck über dem Temperungsdruck, jedoch von nicht mehr als 50 psi (206,845 kPa), und bei einer Temperatur, die nicht niedriger als die zweite Temperatur und nicht höher als die erste Temperatur ist, über einen Zeitraum umfaßt, um den Ballon bei einem Aufblasdruck von 3 at mit einem Enddurchmesser zu versehen, der größer als der zweite Durchmesser ist, jedoch nicht mehr als 90 % des ersten Durchmessers beträgt.

2. Verfahren nach Anspruch 1, wobei der zweite Druck nicht mehr als 20 psi (137,895 kPa) beträgt.

3. Verfahren nach Anspruch 2, wobei der zweite Druck im Bereich von 1 - 10 psi (6,89476 - 68,9476 kPa) liegt.

4. Verfahren nach einem der Ansprüche 1 bis 3, wobei die erste Temperatur im Bereich von 90° - 100° liegt und die zweite Temperatur im Bereich von 70° - 100° liegt.

5. Verfahren nach einem der Ansprüche 1 bis 4, wobei das Blockcopolymer aus weichen Segmenten eines Polyethers und aus harten Segmenten eines Polyesters oder eines Polyamids besteht.

6. Verfahren nach Anspruch 5, wobei die weichen Segmente des Polyethers Polyether von C₂-C₁₀ -Diolen sind.

7. Verfahren nach Anspruch 5 oder 6, wobei die harten Segmente Polyester einer aromatischen Dicarbonsäure und eines C₂-C₄ -Diols sind.

8. Verfahren nach Anspruch 5 oder 6, wobei die harten Segmente Polyamide sind, die ausgewählt sind aus der Gruppe bestehend aus C₆- oder höheren Carbonsäuren und C₆- oder höheren organischen Diaminen und C₆- oder höheren aliphatischen ω-Amino-α-säuren.

9. Verfahren nach einem der vorhergehenden Ansprüche, wobei:
der erste Blasdruck über dem Umgebungsdruck liegt und höher als 50. psi (206,843 kPa) bei der ersten Temperatur ist, um den Ballon bei einem Aufblasdruck von 3 at mit dem ersten Durchmesser auszubilden,
die zweite Temperatur über der Umgebungstemperatur liegt und niedriger als die erste Temperatur oder gleich derselben ist, und bei einem zweiten Druck, der im Bereich von 0-20 psi (0 - 137,895 kPa) über einen Zeitraum liegt, der ausreicht, um den ausgebildeten Ballon bei einem Aufblasdruck von 3 atm auf den zweiten Durchmesser zu schrumpfen, der kleiner als 90 % des ersten Durchmessers ist.

10. Verfahren nach Anspruch 9, wobei der Ballon in der Form des festen Durchmessers über einen Zeitraum derart unter Druck gesetzt wird, daß der Enddurchmesser 85 % oder weniger des ersten Durchmessers beträgt.

11. Verfahren nach Anspruch 9, wobei der Ballon in der Form des festen Durchmessers über einen Zeitraum derart unter Druck gesetzt wird, daß der Enddurchmesser 65 - 75 % des ersten Durchmessers beträgt.

## Revendications

1. Procédé de formation d'un ballonnet destiné à un appareil médical, dans lequel un tube d'un matériau de polymère thermoplastique est radialement dilaté sous une première pression à une première température pour former le ballonnet à un premier diamètre, et le procédé comprenant l'étape supplémentaire consistant à recuire le ballonnet à une seconde température au-dessus de la température ambiante et inférieure à la première température et à une seconde pression suffisamment longtemps pour rétrécir le ballonnet formé à un second diamètre inférieur au premier diamètre,
**caractérisé en ce que**
- le matériau de polymère thermoplastique est un matériau de copolymère séquencé,
- la seconde pression est au-dessus de la pression ambiante et inférieure à la première pression, et
- le procédé comprenant en outre l'étape consistant à mettre sous pression le ballonnet sous une forme de diamètre fixé, ledit diamètre fixé étant supérieur audit second diamètre mais ne dépassant pas 90 % dudit premier diamètre, à une pression au-dessus de la pression de recuit mais ne dépassant pas 50 psi (206,843 kPa) et à une température qui n'est pas inférieure à ladite seconde température et qui n'est pas supérieure à ladite première température, pendant un certain temps, afin de procurer au ballonnet un diamètre final, à une pression de gonflage de 3 atmosphères, qui est supérieur audit second diamètre mais qui ne dépasse pas 90 % dudit premier diamètre.

2. Procédé selon la revendication 1, dans lequel la seconde pression n'est pas supérieure à 20 psi (137,895 kPa).

3. Procédé selon la revendication 2, dans lequel la seconde pression se situe dans la plage de 1 à 10 psi (6,89476 à 68,9476 kPa).

4. Procédé selon l'une des revendications 1 à 3, dans lequel la première température se situe dans la plage de 90 à 100 °C et la seconde température se situe dans la plage de 70 à 100 °C.

5. Procédé selon l'une des revendications 1 à 4, dans lequel le copolymère séquencé est constitué de segments mous d'un polyéther et de segments durs d'un polyester ou d'un polyamide.

6. Procédé selon la revendication 5, dans lequel les segments mous de polyéther sont des polyéthers de diols en C₂ à C₁₀.

7. Procédé selon la revendication 5 ou 6, dans lequel les segments durs sont des polyesters d'un acide dicarboxylique aromatique et de diols en C₂ à C₄.

8. Procédé selon la revendication 5 ou 6, dans lequel les segments durs sont des polyamides choisis parmi le groupe constitué d'une combinaison d'acides carboxyliques en C₆ ou plus, de diamines organiques en C₆ ou plus et de ω-amino-α-acides aliphatiques en C₆ ou plus.

9. Procédé selon l'une des revendications précédentes, dans lequel :
la première pression de gonflage se trouve au-dessus de la pression ambiante et supérieure à 50 psi (206,843 kPa) à la première température pour former le ballonnet pour qu'il présente le premier diamètre à une pression de gonflage de 3 atmosphères,
la seconde température se trouve au-dessus de la température ambiante et inférieure ou égale à la première température et à une seconde pression qui est dans la plage de 0 à 20 psi (0 à 137,895 kPa) pendant un temps suffisant pour rétrécir le ballonnet formé pour qu'il présente le second diamètre à une pression de gonflage de 3 atmosphères qui est inférieur à 90 % du premier diamètre.

10. Procédé selon la revendication 9, dans lequel le ballonnet est mis sous pression sous la forme du diamètre fixé pendant un temps tel que ledit diamètre final soit de 85 % ou moins dudit premier diamètre.

11. Procédé selon la revendication 9, dans lequel le ballonnet est mis sous pression sous la forme du diamètre fixé pendant un temps tel que ledit diamètre final soit de 65 à 75 % dudit premier diamètre.
